**Europäisches Patentamt**

**European Patent Office**

**Office européen des brevets**

(19)

(11) Publication number: **0 009 290**
**B1**

(12) # EUROPEAN PATENT SPECIFICATION

(45) Date of publication of patent specification: **07.07.82**

(51) Int. Cl.³: **C 07 D 209/52**

(21) Application number: **79200519.1**

(22) Date of filing: **17.09.79**

(54) 3-Azabicyclo(3.1.0)hexane derivatives and process for their preparation.

(30) Priority: **27.09.78 GB 3833178**

(43) Date of publication of application:
**02.04.80 Bulletin 80/7**

(45) Publication of the grant of the patent:
**07.07.82 Bulletin 82/27**

(84) Designated Contracting States:
**AT BE CH DE FR GB IT NL SE**

(56) References cited:
**DE - A1 - 2 641 295**
**GB - A - 2 015 515**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Mason, Ronald Frank**
**"Kingswood" Westwell**
**Near Ashford Kent (GB)**
Inventor: **Wood, Derek Alexander**
**76 Sterling Road**
**Sittingbourne Kent (GB)**

(74) Representative: **Rogers, Roy Charles et al,**
**4 York Road**
**London SE1 7NA (GB)**

Courier Press, Leamington Spa, England.

## 3-Azabicyclo[3.1.0]hexane derivatives and process for their preparation

The present invention relates to novel derivatives of 2 - cyano - 3 - azabicyclo[3.1.0]hexane. The invention also relates to a process for the preparation of the said novel derivatives.

2 - Cyano - 3 - azabicyclo[3.1.0]hexane is an interesting compound which can be conveniently used as starting material for the preparation of 2 - carboxy - 3 - azabicyclo[3.1.0]hexane or derivatives thereof, which exhibit biological properties, especially with respect to the sterilisation of male anthers in plants, in particular in small grain cereals.

It will be appreciated that 2 - cyano - 3 - azabicyclo[3.1.0]hexane exhibits both optical and geometric isomerism. Geometric isomerism with respect to the cyano group is of the cis/trans nature reflecting the mutual positions of the 2 - cyano group and the 3,4 - methano bridging group.

Mixtures of geometric isomers are usually difficult to handle and to formulate, as the different isomers have different physical properties. In addition, it is possible that one isomer will exhibit more biological activity and/or selectivity than its geometric isomer. Thus there is a need to separate geometric isomers when they have been produced by methods which lead to the formation of both isomers. It is also advantageous to obtain a cis/trans separation in the event that further synthesis requires a specific isomer as the starting material.

It has now been found that the benzene sulphonic acid and toluene sulphonic acid salts of 2 - cyano - 3 - azabicyclo[3.1.0]hexane, which are novel compounds, are very useful intermediates for the separation of the cis and trans isomers of the cyano compounds.

The invention therefore provides these salts, and a process for their preparation which comprises treating 2 - cyano - 3 - azabicyclo[3.1.0]hexane with benezene sulphonic acid or a toluene sulphonic acid.

It should be understood that a salt according to the invention may be a cis isomer, a trans isomer or a mixture thereof.

Preferably the salt is a p-toluene sulphonic acid salt.

The cis and trans sulphonic acid salts according to the invention have very different solubilities in water, methanol or ethanol, or mixtures of two or all of these solvents, the cis salts being very much more soluble than the trans salts. Therefore in a preferred embodiment of the process according to the invention for the preparation of a sulphonic acid salt, a mixture of the cis and trans isomers of 2 - cyano - 3 - azabicyclo[3.1.0]hexane is treated with benzene sulphonic acid or a toluene sulphonic acid, preferably p-toluene sulphonic acid, and the resulting cis and trans salts are separated by virtue of their different solubilities in water and/or methanol and/or ethanol.

Suitable separation methods include producing solid mixed salts, followed by preferential dissolving the cis salt, as well as producing a solution of mixed salts followed by precipitating out the trans salt.

The conversion of the isomeric nitriles into the corresponding sulphonic acid salts is preferably carried out under conditions which cause rapid formation of a solid mass of mixed salts. This will normally be achieved when a mixture of the nitriles in a suitable solvent, for example a lower alkanol such as methanol, ethanol or isopropanol, is treated with a substantially equimolar amount of benzene sulphonic acid or a toluene sulphonic acid in a solvent, preferably in the same solvent as the solvent for the nitriles. If desired, solvent mixtures may be used, e.g. mixtures comprising a lower alkanol and a substantial amount of a liquid alkane such as pentane, hexane or octane.

The separation of the cis isomer form the mixture may then be achieved by extracting the solid mass of mixed salts with water, and/or methanol and/or ethanol, preferably with water chilled to a temperature below 5°C, or with hot ethanol.

The dissolved cis sulphonic acid salt can be removed from the virtually undissolved trans sulphonic acid salt by conventional methods such as decantation or filtration.

It has been observed that if the reaction is carried out in the presence of large quantities of methanol and/or ethanol as solvent the precipitation of only trans isomer, rather than mixed isomers, is promoted. The precipitated trans isomer may then be separated off.

When extracting cis isomer from mixed solid salts, it is preferred to use the minimum amount of water and/or methanol and/or ethanol in order to minimise the dissolution of the trans salt. It may be advantageous to perform a number of extractions until a constant cis/trans ratio in the separated salts has been obtained. The substantially insoluble trans isomer can usually be obtained having an isomeric purity of at least 85%, often in excess of 95%. Removal of solvent from the filtrate will give the cis salt.

The formation of the sulphonic acid salts may be carried out in the additional presence of large quantities of water or water containing a small amount of a lower alkanol. A precipitate will thus normally not be formed at all. If small quantities of water are used, the trans isomer may crystallise at once leaving the cis isomer in solution, which facilitates the separation of the isomeric salts.

In the absence of a precipitate, evaporation of the aqueous solvent from the solution obtained will initially lead to the formation of solid trans isomer (which can be isolated as such) while continued evaporation will lead to the formation of solid mixed salts which can then be extracted with water and/or methanol and/or ethanol to effect separation.

The process according to the present invention also has the advantage that any non-basic as well as soluble basic impurites present my be discarded during the separation process, thus achieving a simultaneous chemical purification step.

Mixtures of the cis and trans isomers of 2 - cyano - 3 - azabicyclo[3.1.0]hexane may be prepared by the reaction of 2 - cyano - 3 - azabicyclo[3.1.0]hexan - 4 - one with triethyloxonium tetrafluoroborate and subsequent reaction with sodium borohydride, as described in European Patent Application No. 79200096.

Mixtures of nitriles which have been obtained by partially converting an unwanted isomer by the isomerisation process as described and claimed in European Patent Application 79200518 can be used. Thus when trans 2 - cyano - 3 - azabicyclo[3.1.0]hexane is isomerised according to the process described and claimed in said co-pending application, a mixture of cis and trans isomers is obtained which normally contains at least 60—65% of the cis isomer. If desired, isomer mixtures comprising a relatively low amount of the cis isomer, e.g. amounts in the range of from 5 to 40% by weight of total mixture, can be used in the process according to the present invention.

If desired, after separation of the cis and trans salts, either or both salts can be converted into the corresponding cis or trans 2 - cyano - 3 - azabicyclo[3.1.0]hexane or the corresponding cis or trans 2 - carboxy - 3 - azabicyclo[3.1.0]hexane or a salt and/or an ester, by methods analogous to known methods. For example, hydrolysis of the salt using a strong acid, for example hydrochloric acid, followed by removal of the free sulphonic acid over an ion exchange column, gives the corresponding isomer of 2 - carboxy - 3 - azabicyclo[3.1.0]hexane, which can if desired be converted into a salt and/or ester thereof by known methods.

The present invention has not only the advantage that it provides a method for the separation of 2 - cyano - 3 - azabicyclo[3.1.0]hexane isomers with high isomeric purity, but if desired, it also allows a partial isomerisation when heating the cis or trans sulphonic acid salt (or a mixture comprising predominantly cis or trans sulphonic acid salt) in water or a lower alkanol. This may be useful if it is desired to obtain cis isomer starting from trans 2 - cyano - 3 - azabicyclo[3.1.0]-hexane - 2 p-toluene sulphonic acid salt or from a mixture having a relatively high content of the trans isomer. Heating the trans isomer (e.g. containing 95% by weight trans isomer) in water and/or a lower alkanol, optionally in the presence of compound containing, or capable under the isomerisation conditions of generating, free cyanide moieties results in a mixture comprising about two-thirds of cis and about one-third of trans isomer. On cooling a filtrate containing predominantly cis 2 - cyano - 3 - azabicyclo[3.1.0]hexane p-toluene sulphonic acid salt will be obtained. The remaining insoluble trans salt can be recycled and subjected to further isomerisation treatment.

The invention is illustrated by the following Example.

Example
(a) Preparation of mixed salts

To a mixture of 33 g (0.31 M) cis and trans 2 - cyano - 3 - azabicyclo[3.1.0]hexane (cis/trans ratio 62/38) in isopropanol/hexane (50/50 bw) was added slowly a solution of 57 g of p-toluene sulphonic acid (99% min. pur., 0.3 M) in isopropanol (0.5 litre) whilst keeping the temperature at 15—25°C by external cooling.

The mixed salts readily crystallised. The resulting slurry was diluted with hexane (0.5 litre) and cooled to 0°C. The mixed salts were filtered and washed with 50/50 isopropanol/hexane (1 litre) and finally with some hexane. Air dried salts weighed 72 g (0.26 M) equivalent to 85% yield.

(b) Separation of mixed salts

The mixture obtained under (a) was slurried with demineralised water at 0°C followed by filtering. 25.5 g of solid product was obtained after drying to constant weight and contained 95+% isomerically pure trans 2 - cyano - 3 - azabicyclo[3.1.0]hexane p-toluene sulphonic acid salt. This compound was characterised by comparison with a sample of trans 2 - cyano - 3 - azabicyclo[3.1.0]hexane p-toluene sulphonic acid salt obtained from mixing a small amount of trans 2 - cyano - 3 - azabicyclo[3.1.0]hexane in ethanol with a solution of an equimolar amount of p-toluene sulphonic acid monohydrate in ethanol. Filtration and recrystallisation from methanol gave trans 2 - cyano - 3 - azabicyclo[3.1.0]hexane p-toluene sulphonic acid salt m.p. 159—161°C (dec.). The all-trans structure was confirmed by derivatisation gas-liquid chromatography and NMR analysis.

Analysis
Calculated for $C_{13}H_{16}O_3N_2S$
   C 55.7   H 5.8   N 10.0%
Found
   C 55.4   H 5.6   N 9.9%

Concentrating the filtrate until crystals just started to separate followed by cooling in iced water and filtration gave a further amount of trans 2 - cyano - 3 - azabicyclo[3.1.0]hexane p-toluene sulphonic acid salt (5 g).

The filtrate contained cis 2 - cyano - 3 - azabicyclo[3.1.0]hexane p-toluene sulphonic acid salt (cis/trans ration 92/8), which afforded after evaporation, recrystallisation from isopropanol and drying *in vacuo*, cis 2 - cyano - 3 - azabicyclo[3.1.0]hexane *p*-toluene sulphonic acid salt m.p. 126—128°C (slight dec.). The cis/trans ratio was 99/1 (derivatisation, gas liquid chromatography).

Analysis

Calculated for $C_{13}H_{16}O_3N_2S$

C 55.7   H 5.8   N 10.0%

Found

C 55.3   H 5.5   N 9.7%

(c) Conversion of separated salts into the corresponding acids

The filtrate obtained from (b) was treated with concentrated hydrochloric acid (1.5 litre) and the mixture boiled under nitrogen. The resulting solution was stripped on a rotatory evaporator and the resulting semi-solid redissolved in demineralised water (1.8 litre).

The solution comprising *p*-toluene sulphonic acid, hydrochloric acid and product was then charged to a Dowex 50 $H^+$ cation exchange column which after washing through the mineral acids released the product by elution with aqueous ammonia, removal of water at reduced pressure and crystalisation from isopropanol. After drying, the product cis 2 - carboxy - 3 - azabicyclo[3.1.0]hexane was obtained with a water content of 10.1% and chemical purity 94+% in a cis/trans ratio 91/9 (m.p. 249—250°C (dec.)). The chemical yield for the hydrolysis step calculates to 77% based on the starting mixed salts.

Trans 2 - carboxy - 3 - azabicyclo[3.1.0]-hexane was obtained by a similar hydrolysis procedure from trans 2 - cyano - 3 - azabicyclo[3.1.0]hexane *p*-toluene sulphonic acid salt. The product obtained contained less than 1% bw of water and its isomeric purity exceeded 95%. The chemical yield was 82% (m.p. 260—262°C (dec.)).

**Claims for the contracting states: BE, CH, DE, FR, GB, IT, NL, SE**

1. A benzene sulphonic acid or toluene sulphonic acid salt of 2 - cyano - 3 - azabicyclo[3.1.0]hexane.

2. A *p*-toluene sulphonic acid salt of 2 - cyano - 3 - azabicyclo[3.1.0]hexane.

3. A process for the preparation of a compound as claimed in Claim 1, characterised in that 2 - cyano - 3 - azabicyclo[3.1.0]-hexane is treated with benzene sulphonic acid or a toluene sulphonic acid.

4. A process as claimed in Claim 3, characterised in that the starting 2 - cyano - 3 - azabicyclo[3.1.0]hexane is a mixture of trans and cis isomers and that the resulting trans and cis salts are separated from each other by virtue of the different solubilities of the isomeric salts in water, and/or methanol and/or ethanol.

5. A process as claimed in Claim 4, characterised in that a solution of the trans and cis 2 - cyano - 3 - azabicyclo[3.1.0]hexane in a solvent is treated with a substantially equimolar amount of the sulphonic acid in the same solvent to produce a precipitate of trans and cis salts, and that subsequently cis salt is extracted from the precipitate with water and/or methanol and/or ethanol.

6. A process as claimed in Claim 5, characterised in that as solvent a lower alkanol optionally in admixture with a liquid alkane is applied.

7. A process as claimed in either Claim 5 or Claim 6, characterised in that the cis salt is extracted using water at a temperature below 5°C or hot ethanol.

8. A process as claimed in any one of Claims 4 to 7, characterised in that it comprises the further step after separation of the cis salt from the trans salt, of converting either the cis salt or the trans salt or both into the corresponding cis or trans 2 - cyano - 3 - azabicyclo[3.1.0]-hexane or into the corresponding cis or trans 2 - carboxy - 3 - azabicyclo[3.1.0]hexane or a salt and/or an ester thereof.

**Claims for the contracting state: AT**

1. A process for the preparation of a benzene sulphonic acid or toluene sulphonic acid salt of 2 - cyano - 3 - azabicyclo[3.1.0]hexane, characterised in that 2 - cyano - 3 - azabicyclo[3.1.0]hexane is treated with benzene sulphonic acid or a toluene sulphonic acid.

2. A process as claimed in claim 1, characterised in that the starting 2 - cyano - 3 - azabicyclo[3.1.0]hexane is a mixture of trans and cis isomers and that the resulting trans and cis salts are separated from each other by virtue of the different solubilities of the isomeric salts in water, and/or methanol and/or ethanol.

3. A process as claimed in claim 2, characterised in that a solution of the trans and cis 2 - cyano - 3 - azabicyclo[3.1.0]hexane in a solvent is treated with a substantially equimolar amount of the sulphonic acid in the same solvent to produce a precipitate of trans and cis salts, and that subsequently cis salt is extracted from the precipitate with water and/or methanol and/or ethanol.

4. A process as claimed in claim 3, characterised in that as solvent a lower alkanol optionally in admixture with a liquid alkane is applied.

5. A process as claimed in either claim 3 or claim 4, characterised in that the cis salt is extracted using water at a temperature below 5°C or hot ethanol.

6. A process as claimed in any one of claims 2 to 5, characterised in that it comprises the further step after separation of the cis salt from the trans salt, of converting either the cis salt or the trans salt or both into the corresponding cis or trans 2 - cyano - 3 - azabicyclo[3.1.0]-hexane or into the corresponding cis or trans

2 - carboxy - 3 - azabicyclo[3.1.0]hexane or a salt and/or an ester thereof.

## Patentansprüche für die Vertragsstaaten: BE, CH, DE, FR, GB, IT, NL, SE

1. Ein Benzolsulfonsäure- oder Toluolsulfonsäuresalz von 2 - Cyano - 3 - azabicyclo[3.1.0]hexan.

2. Ein p-Toluolsulfonsäuresalz von 2 - Cyano - 3 - azabicyclo[3.1.0]hexan.

3. Ein Verfahren zur Herstellung einer Verbindung nach Anspruch 1, dadurch gekennzeichnet, dass 2 - Cyano - 3 - azabicyclo-[3.1.0]hexan mit Benzolsulfonsäure oder mit einer Toluolsulfonsäure behandelt wird.

4. Verfahren nach Anspruch 3, dadurch gekennzeichnet, dass das Ausgangs - 2 - cyano - 3 - azabicyclo[3.1.0]hexan ein Gemisch aus transund cis-Isomeren ist und dass die gebildeten trans- und cis-Salze voneinander aufgrund der verschiedenen Löslichkeiten der isomeren Salze in Wasser und/oder Methanol und/oder Ethanol getrennt werden.

5. Verfahren nach Anspruch 4, dadurch gekennzeichnet, dass eine Lösung des trans- und cis - 2 - Cyano - 3 - azabicyclo[3.1.0]hexans in einem Lösungsmittel mit einer im wesentlichen äquimolaren Menge der Sulfonsäure in dem gleichen Lösungsmittel behandelt wird, um einen Niederschlag von trans- und cis-Salzen zu ergeben, und dass hierauf das cis-Salz aus dem Niederschlag mit Wasser und/oder Methanol und/oder Ethanol extrahiert wird.

6. Verfahren nach Anspruch 5, dadurch gekennzeichnet, dass als Lösungsmittel ein niederes Alkanol, gewünschtenfalls im Gemisch mit einem flüssigen Alkan, angewendet wird.

7. Verfahren nach Anspruch 5 oder 6, dadurch gekennzeichnet, dass das cis-Salz unter Verwendung von Wasser bei einer Temperature von unter 5°C oder unter Verwendung von heissem Ethanol extrahiert wird.

8. Verfahren nach einem der Ansprüche 4 bis 7, dadurch gekennzeichnet, dass es nach der Abtrennung des cis-Salzes vom trans-Salz die weitere Stufe der Umwandlung von entweder dem cis-Salz oder dem trans-Salz oder von beiden in das entsprechende cis-oder trans - 2 - Cyano - 3 - azabicyclo[3.1.0]haxan oder in das entsprechende cis- oder trans - 2 - Carboxy - 3 - azabicyclo[3.1.0]hexan oder in ein Salz und/oder einen Ester hiervon umfasst.

## Patentansprüche für den Vertragsstaat: AT

1. Verfahren zur Herstellung eines Benzolsulfonsäure- oder Toluolsulfonsäuresalzes von 2 - Cyano - 3 - azabicyclo[3.1.0]hexan, dadurch gekennzeichnet, dass 2 - Cyano - 3 - azabicyclo[3.1.0]hexan mit Benzolsulfonsäure oder mit einer Toluolsulfonsäure behandelt wird.

2. Verfahren nach Anspruch 1, dadurch gekennzeichnet, dass das Ausgangs - 2 - cyano - 3 - azabicyclo[3.1.0]hexan ein Gemisch aus trans- und cis-Isomeren ist und dass die gebildeten trans- und cis-Salze voneinander aufgrund der verschiedenen Löslichkeiten der isomeren Salze in Wasser und/oder Methanol und/oder Ethanol getrennt werden.

3. Verfahren nach Anspruch 2, dadurch gekennzeichnet, dass eine Lösung des trans- und cis - 2 - Cyano - 3 - azabicyclo[3.1.0]hexans in einem Lösungsmittel mit einer im wesentlichen äquimolaren Menge der Sulfonsäure in dem gleichen Lösungsmittel behandelt wird, um einen Niederschlag von trans- und cis-Salzen zu ergeben, und dass hierauf das cis-Salz aus dem Niederschlag mit Wasser und/oder Methanol und/oder Ethanol extrahiert wird.

4. Verfahren nach Anspruch 3, dadurch gekennzeichnet, dass als Lösungsmittel ein niederes Alkanol, gewünschtenfalls im Gemisch mit einem flüssigen Alkan, angewendet wird.

5. Verfahren nach Anspruch 3 oder 4, dadurch gekennzeichnet, dass das cis-Salz unter Verwendung von Wasser bei einer Temperatur von unter 5°C oder unter Verwendung von heissem Ethanol extrahiert wird.

6. Verfahren nach einem der Ansprüche 2 bis 5, dadurch gekennzeichnet, dass es nach der Abtrennung des cis-Salzes vom trans-Salz die weitere Stufe der Umwandlung von entweder dem cis-Salz oder dem trans-Salz oder von beiden in das entsprechende cis-oder trans - 2 - Cyano - 3 - azabicyclo[3.1.0]hexan oder in das entsprechende cis- oder trans - 2 - Carboxy - 3 - azabicyclo[3.1.0]hexan oder in ein Salz und/oder einen Ester hiervon umfasst.

## Revendications pour les etats contractants: BE, CH, DE, FR, GB, IT, NL, SE

1. Un sel d'acide benzène-sulfonique ou d'acide toluène-sulfonique du 2 - cyano - 3 - azabicyclo(3.1.0)hexane.

2. Un sel d'acide p-toluène-sulfonique du 2 - cyano - 3 - azabicyclo(3.1.0)hexane.

3. Un procédé pour la préparation d'un composé tel que défini dans la revendication 1, caractérisé en ce qu'on traite le 2 - cyano - 3 - azabicyclo(3.1.0)hexane per l'acide benzène-sulfonique ou par un acide toluène-sulfonique.

4. Un procédé selon la revendication 3, caractérisé en ce que le 2 - cyano - 3 - azabicyclo(3.1.0)hexane de départ est un mélange d'isomères cis et trans et que les sels cis et trans résultants sont séparés l'un de l'autre grâce aux solubilités différentes des sels isomères dans l'eau et/ou le méthanol et/ou l'éthanol.

5. Un procédé selon la revendication 4, caractérisé en ce qu'une solution du trans et cis 2 - cyano - 3 - azabicyclo(3.1.0)hexane dans un solvant est traitée par une quantité sensiblement équimolaire de l'acide sulfonique dans le même solvant de façon à produire un précipité de sels cis et trans, et qu'ensuite le sel cis est extrait du précipité au moyen d'eau et/ou de méthanol et/ou d'éthanol.

6. Un procédé selon la revendication 5, caractérisé en ce que comme solvant, on utilise un alcanol inférieur éventuellement en mélange avec un alcane liquide.

7. Un procédé selon l'une des revendications 5 et 6, caractérisé en ce qu'on extrait le sel cis en utilisant de l'eau à une température plus basse que 5°C ou de l'éthanol chaud.

8. Un procédé selon l'une quelconque des revendications 4 à 7, caractérisé en ce qu'il comprend l'étape supplémentaire, après qu'on a séparé le sel cis du sel trans, consistant à transformer le sel cis ou le sel trans ou les deux en cis ou trans 2 - cyano - 3 - azabicyclo(3.1.0)- hexane correspondant ou en cis ou trans 2 - carboxy - 3 - azabicyclo(3.1.0)hexane correspondant ou en un sel et/ou un ester de ces composés.

**Revendications pour l'etat contractant: AT**

1. Un procédé pour la préparation d'un sel d'acide benzènesulfonique ou d'acide toluène-sulfonique du 2 - cyano - 3 - azabicyclo-(3.1.0)hexane, caractérisé en ce qu'on traite le 2 - cyano - 3 - azabicyclo(3.1.0)hexane par l'acide benzènesulfonique ou par un acide toluène-sulfonique.

2. Un procédé selon la revendication 1, caractérisé en ce que le 2 - cyano - 3 - azabi-cyclo(3.1.0)hexane de départ est un mélange d'isomères cis et trans et que les sels cis et trans résultants sont séparés l'un de l'autre grâce aux solubilites différentes des sels isomères dans l'eau et/ou le méthanol et/ou l'éthanol.

3. Un procédé selon la revendication 2, caractérisé en ce qu'une solution du trans et cis 2 - cyano - 3 - azabicyclo(3.1.0)hexane dans un solvant est traitée par une quantité sensiblement équimolaire de l'acide sulfonique dans le même solvant de manière à produire un précipité de sels cis et trans, et qu'ensuite le sel cis est extrait du précipité au moyen d'eau et/ou de méthanol et/ou d'éthanol.

4. Un procédé selon la revendication 3, caractérisé en ce que comme solvant on utilise un alcanol inférieur éventuellement en mélange avec un alcane liquide.

5. Un procédé selon l'une des revendications 3 et 4, caractérisé en ce qu'on extrait le sel cis en utilisant de l'eau à une température plus basse que 5°C ou de l'éthanol chaud.

6. Un procédé selon l'une quelconque des revendications 2 à 5, caractérisé en ce qu'il comprend l'étape supplémentaire, après qu'on a séparé le sel cis du sel trans, consistant à transformer le sel cis ou le sel trans ou les deux en cis ou trans 2 - cyano - 3 - azabicyclo(3.1.0)- hexane correspondant ou en cis ou trans 2 - carboxy - 3 - azabicyclo(3.1.0)hexane correspondant ou en un sel et/ou un ester de ces composés.